# EUROPEAN PATENT SPECIFICATION

(11) **EP 3 233 040 B1**
(45) Date of publication and mention of the grant of the patent: **22.05.2019**
(21) Application number: 15808628.0
(22) Date of filing: 14.12.2015
(51) Int. Cl.: A61K 8/67, A61Q 19/06, A61K 8/99

(54) **COMPOSITION FOR IMPROVING THE CELLULITE APPEARANCE OF SKIN**
ZUSAMMENSETZUNG ZUR VERBESSERUNG DES CELLULITEERSCHEINUNGSBILDES VON HAUT
COMPOSITION DESTINÉE À L'AMÉLIORATION DE L'ASPECT "PEAU D'ORANGE" DE LA PEAU

(30) Priority: 18.12.2014 FR 1462825
(43) Date of publication of application: 25.10.2017
(73) Proprietor: NUTRICOS Technologies, 92117 Clichy Cedex (FR)
(72) Inventor: PICCARDI, Nathalie, 93400 Saint Ouen (FR); CHENITI, Ashène, 93400 Saint Ouen (FR)
(74) Representative: Lavoix
(86) International application number: PCT/EP2015/079626
(87) International publication number: WO 2016/096747

(56) References cited:
- WO-A1-99/47112
- WO-A1-2006/104730
- FR-A1- 3 001 633

## Description

The present invention relates to the field of dietary supplements and functional foods intended for improving the cellulite appearance of skin.

Cellulite is presented by unevenness of the skin surface when pinched (dimpling) or not (case of more pronounced cellulite). It is a cosmetic problem, affecting 80% to 90% of women, regardless of their body type, to varying degrees. Cellulite does not arise before puberty. It is generally not painful.

In terms of the skin, this phenomenon known as cellulite is conveyed by a lumpy appearance showing signs of "dimpling" in places. In clinical terms, cellulite is conveyed by a modification of the texture of the subcutaneous and surface tissue, characterized in particular by:
- skin which is thicker overall,
- skin with a firmer texture,
- more sensitive skin which may according to the stage of progression of the cellulite be painful on palpation, and/or
- less mobile skin tissue due to the loss of adherence and cohesion of the deep layers of the skin.

Cellulite is particularly located in the pelvic area and the lower limbs (cellulite "saddlebags" or "flabby legs"). These modifications may also give rise to definitive scar deformations.

Dimpling and cellulite are frequently perceived to be distressing, due to their unsightly appearance, by subjects, particularly women, who suffer from the condition particularly when they are not overweight.

In particular in these women, the cellulite appearance is not associated with overweight but with other factors such as hormonal factors or stress. In such subjects, weight loss does not help reduce the cellulite appearance satisfactorily. For this reason, compositions envisaged to reduce the cellulite appearance of the skin of overweight women are not necessarily effective on women of normal weight. Indeed, a clear distinction should be made between obesity and cellulite, even though these two phenomena may coexist. Adiposity is merely the accumulation of fat tissue in empty spaces. When fat tissue exceeds the normal value of 30%, the term obesity is used. Furthermore, cellulite also involves a transformation and alteration of interstitial subcutaneous tissue, and is not merely an accumulation of fat. This confusion compels women to seek to lose weight in order to improve the appearance of their cellulite, at the risk for subjects of normal weight of inducing muscle loss and tissue alteration which may be irrevocable (Bacci and Leibaschoff, Pathophysiology of cellulite pp 41-74, in Cellulite Pathophysiology and treatment, published by MP Goldman et al, 2006 Taylor & Francis ed).

Therefore, there is a significant need for compositions suitable for improving the cellulite appearance of skin, particularly in women of normal weight, and notably women who have not sustained or are not sustaining weight loss following a diet.

Topical treatments for combating "dimpling" are known. However, topical agents are not always active due to the poor skin penetration thereof, at a dermal and hypodermal level. Furthermore, by definition, topical products act locally on the areas to be treated, where they may be unevenly distributed, and require repeated thorough applications. They may in some cases be the cause of skin-related side-effects, or discomfort. Document WO9947112 discloses the topical application of niacinamide for combating cellulite.

By contrast, the oral and/or parenteral route offers the advantage of overall action on the entire skin, in the deep layers thereof (dermis, hypodermis). Indeed, metabolites and further active nutrients are particularly distributed within the dermal matrix via the bloodstream.

Therefore, there is a more specific need for a composition for oral administration suitable for improving the cellulite appearance of skin, particularly in women of normal weight, particularly those who have not sustained or are not sustaining weight loss following a diet.

Surprisingly, the inventors demonstrated that the oral administration of a composition comprising at least one probiotic and at least one B group vitamin has a beneficial activity on skin quality and notably on the cellulite appearance thereof, particularly on the biomechanical properties thereof.

The present invention thus relates to uses, notably cosmetic uses, involving a composition for oral administration comprising at least one probiotic and at least one B group vitamin, particularly for improving the cellulite appearance of skin.

It is understood within the scope of the present invention that the expression "by oral route" covers products administered by the oral route, these products, for example in dietary supplement form as explained hereinafter, producing an effect on the skin in terms of cosmetic appearance and comfort, or for beauty purposes, for example with a view to modifying the appearance thereof and, notably, beautifying same.

The oral route also offers the advantage of a quick and non-restrictive method of administration.

The term "biomechanical properties of the skin" within the scope of the present invention denotes the properties in respect of stretchability, tone, firmness, suppleness, density and/or elasticity of the skin.

The term "prevent" with regard to a cosmetic defect of the skin, within the scope of the present invention denotes the act of reducing the risk of occurrence of this defect.

The term "cellulite appearance of skin" denotes the external appearance of the skin and its modifications induced by cellulite, such as for example fat deposits or "dimpling" which may be more or less localized in specific areas such as the thighs, buttocks, arms or abdomen.

The term "improve the cellulite appearance of skin" denotes herein more specifically reducing the number and depth of the visible depressions caused by cellulite, improving the clinical appearance of lesions caused by cellulite, reducing flaccidity and lack of firmness of the skin caused by cellulite, reducing the degree of cellulite. This may include reducing the dimpling appearance, increasing skin uniformity, smoothing depressions, reducing the visibility of cellulite and increasing softness of the skin notably on the thighs and buttocks.

According to one preferred embodiment, the invention is particularly suitable for reinforcing the biomechanical properties of the skin, particularly for combating slack, flabby, distended, saggy skin, fatty deposits, and/or reinforcing and/or restoring skin elasticity or firmness.

According to a further preferred embodiment, the invention is suitable for preventing, treating or reducing a cosmetic skin defect associated with cellulite chosen from loss of firmness, loss of elasticity, loss of density, loss of tone of the skin, the presence and/or visibility and/or size of the fatty deposit(s), sagging skin, or slackening of the skin.

A composition, notably a cosmetic composition, used within the scope of the invention comprises at least one probiotic and at least one B group vitamin.

The term "probiotics" or "probiotic micro-organisms" denotes microbial cell preparations or components of microbial cells that have a beneficial effect on the well-being of the host (Salminen S, Ouwehand A. Benno Y. et al, " Probiotics: how should they be defined " Trends Food Sci. 1999, 10:107-10). A probiotic micro-organism used within the scope of the invention may be used in a live, semi-activated, inactivated or dead form. In particular, a probiotic micro-organism used within the scope of the invention may be used in a live or inactivated form.

In the sense of the invention, an "inactivated" micro-organism is a micro-organism which is no longer capable, temporarily or definitively, of forming colonies in culture.

In the sense of the invention, a "dead" micro-organism is a micro-organism which is no longer capable, definitively, of forming colonies in culture. The dead or inactivated micro-organisms may have intact or ruptured cell membranes. As such, the term "inactivated" also denotes micro-organism extracts and lysates.

An inactivated probiotic micro-organism suitable for the invention may be prepared by irradiation, heat treatment or by freeze-drying a preparation of probiotic micro-organisms. These methods are known to those skilled in the art.

A probiotic micro-organism used within the scope of the invention may be used in whole form, i.e. essentially in the native form thereof, or in the form of extracts or lysates comprising fractions and/or metabolites of this micro-organism.

According to one embodiment, a probiotic micro-organism suitable for the invention may be of the Lactobacillus genus and preferably of the species *Lactobacillus rhamnosus.*

*Lactobacillus rhamnosus* is among the probiotics isolated from human microflora.

A *Lactobacillus rhamnosus* suitable for the invention may be chosen from among *Lactobacillus rhamnosus* ATCC53103 and *Lactobacillus rhamnosus* NCC 4007 (also referred to as CGMCC 1.3724). These *Lactobacillus rhamnosus* strains are available from the CGMCC (China General Microbiological Culture Collection), ATCC (American Type Culture Collection) and NCC (Nestle Culture Collection) collections, respectively. Preferably, the probiotic used within the scope of the invention is the strain *Lactobacillus rhamnosus* NCC 4007.

*Lactobacillus rhamnosus* can be cultured and prepared for dietary processing according to any method known to those skilled in the art, or it may be obtained commercially in a form suitable for oral administration.

Preferably, the composition used within the scope of the invention comprises 1x10⁵ to 1x10¹² cfu (colony-forming units), preferably 1x10⁷ to 1x10¹¹ cfu of *Lactobacillus rhamnosus,* more preferably 1x10⁹ cfu of *Lactobacillus rhamnosus.*

According to one preferred embodiment, the composition used within the scope of the invention further comprises a further probiotic micro-organism, preferably of the Bifidobacterium genus and particularly of the species *Bifidobacterium longum.*

*Bifidobacterium Iongum* includes the sub-species *Bifidobacterium longum* subsp. *infantis, Bifidobacterium longum* subsp. *longum, and Bifidobacterium longum* subsp. *suis.* Examples of *suitable Bifidobacterium longum* strains include the strains *Bifidobacterium longum* subsp. *longum* BOR1, *Bifidobacterium longum* subsp. *longum* DJ010A, *Bifidobacterium longum* subsp. *longum* JDM301, *Bifidobacterium longum subsp. longum* M58739, *Bifidobacterium longum* subsp. *infantis* 157F-NC, *Bifidobacterium longum* subsp. *infantis* ATCC 15697, *Bifidobacterium longum* subsp. *infantis* ATCC 55813, *Bifidobacterium longum* subsp. *infantis* CCUG 52486, *Bifidobacterium longum* subsp. *infantis* JCM1217, *Bifidobacterium longum* subsp. *infantis JCM1222, Bifidobacterium longum* ATCC BAA-999, *Bifidobacterium longum* FERM BP-7787, *Bifidobacterium longum* ATCC 15707, *Bifidobacterium longum* ATCC 15708, *Bifidobacterium longum* ATCC 55817, *Bifidobacterium longum* FERM P-6548, *Bifidobacterium longum* CNCM 1-1228, the strain *Bifidobacterium longum* deposited under the Budapest Treaty with Collection Nationale de Cultures de Microorganismes (CNCM, Institut Pasteur, 28 rue du Dr Roux, 75724 Paris Cedex 15, France) under the number CNCM I-2170, and the strain *Bifidobacterium longum* BB536. Preferably, the Bifidobacterium longum strain used within the scope of the invention is the strain *Bifidobacterium longum* BB999

Preferably, the composition used within the scope of the invention comprises 1x10⁵ to 1x10¹² cfu, preferably from 1x10⁷ to 1x10¹¹ cfu of *Bifidobacterium longum.*

The vitamins included in the compositions used within the scope of the invention are chosen from among B group vitamins, and are more particularly vitamins B8 and/or B3. In one particular embodiment, said at least one B group vitamin used within the scope of the invention is a mixture of vitamin B3 and vitamin B8.

Preferably, the composition used within the scope of the invention comprises 1 to 10 mg of at least one B group vitamin, preferably 4 to 7 mg of at least one B group vitamin, preferably 5 to 6 mg of at least one B group vitamin. More preferably, the composition used within the scope of the invention comprises 1 to 10 mg of a mixture of vitamin B3 and vitamin B8, preferably 4 to 7 mg of a mixture of vitamin B3 and vitamin B8, preferably 5 to 6 mg of a mixture of vitamin B3 and vitamin B8. In one particular embodiment, the composition according to the invention comprises 1 to 10 mg of vitamin B3 and/or 15 to 45 µg of vitamin B8, preferably 4 to 7 mg of vitamin B3 and/or 20 to 40 µg of vitamin B8, more preferably 5 to 6 mg of vitamin B3 and/or 25 to 30 µg of vitamin B8.

According to one preferred embodiment, the composition used within the scope of the invention comprises the probiotic *Lactobacillus rhamnosus* NCC 4007 and vitamins B3 and B8.

According to one embodiment, the composition used within the scope of the invention further comprises at least one prebiotic compound.

The term "prebiotic compound" denotes dietary substances intended to promote the growth of probiotic bacteria in the intestine. A prebiotic compound suitable for the invention may be chosen from among oligosaccharides, and optionally contain fructose, glucose, galactose, mannose, xylose, fructo-oligosaccharides and/or inulin, dietary fiber, or mixtures thereof.

According to one preferred embodiment, the prebiotic compound suitable for the invention is a fructo-oligosaccharide.

Fructo-oligosaccharides (FOS) belong to the fructan group. They are natural polymers of D-fructose terminated (or not in the case of oligofructoses derived from inulin) with a D-glucose molecule (linked to a D-fructose molecule by a 1-1' glycosidic bond). The fructose molecules are linked to one another by β(1-2) glycosidic bonds. The number of associated fructose molecules is not greater than 10 in the case of FOS. FOS are complex molecules organized in linear or branched chains, generally linked firmly with the other dietary compounds. The main dietary sources of FOS are vegetables (Jerusalem artichoke, onion, leek, chicory root, garlic, banana, asparagus), some cereals (barley, oats, rye) and even mushrooms (shiitake for example).

Preferably, the composition used within the scope of the invention comprises 50 to 100 mg; preferably 75 to 95 mg, more preferably 80 to 90 mg of FOS.

The FOS suitable for the invention may be from commercial sources.

Preferably, the composition used within the scope of the present invention comprises inulin. The inulin suitable for the invention may be an inulin of plant origin extracted from chicory root (for example Orafti GR supplied by BeneOrafti).

Preferably, the composition used within the scope of the invention comprises 40 to 80 mg, preferably 50 to 65 mg, preferably 55 to 60 mg of inulin.

According to one embodiment, the composition used within the scope of the invention further comprises at least one plant extract.

A plant extract suitable for the invention may be chosen from among phenolic pine extracts, green tea or grape seed extracts, or mixtures thereof.

According to one preferred embodiment, a plant extract suitable for the invention may be a grape seed extract.

According to one embodiment, the grape seed extract is dried Vitis vinifera grape seed extract.

Dried Vitis vinifera grape seed extract (95% pure) is rich in procyanidins, anthocyanidins and leucoanthocyanidins (catechin and epicatechin derivatives), both members of the flavonoid family.

Preferably, the composition used within the scope of the invention comprises 80 to 120 mg of grape seed extract.

According to one preferred embodiment, the composition used within the scope of the invention further comprises at least one additional ingredient liable to have an effect on cellulite by oral route.

The additional ingredients suitable for the invention are nutritional agents which may be chosen from among vitamins, hydrosoluble and insoluble plant fiber, minerals, glucosamine and derivatives thereof, hesperidin, or amino acids. The nutritional agents suitable for the invention may also be chosen from among conjugated linoleic acids (or CLA), licorice extracts, chili pepper extracts, saffron extracts, dairy proteins, plant proteins, L-carnitine, chitosan, guar gum, chromium, garcinia cambogia, Psyllium, yerba mate, guarana, common bean or Phaseolus vulgaris L., nopal fiber or Opuntia Ficus Indica.

According to one particularly preferred embodiment, the composition used within the scope of the invention comprises the probiotic *Lactobacillus rhamnosus* NCC 4007, fructo-oligosaccharides, inulin and a mixture of vitamins B3 and vitamin B8. According to one more preferred embodiment, the composition used within the scope of the invention comprises 1x10⁵ to 1x10¹² cfu of *Lactobacillus rhamnosus* NCC 4007, 4 to 7 mg of a mixture of vitamin B3 and vitamin B8, 50 to 100 mg of FOS and 40 to 80 mg of inulin.

Preferably, the composition used within the scope of the invention comprises, or consists of, *Lactobacillus rhamnosus,* fructo-oligosaccharides, chicory root extracts (*Cichorium intybus*), coating agent (hydroxypropylmethylcellulose), inulin extracted from chicory root (*Cichorium intybus*), anticaking agents (fatty acid magnesium salts, silica), vitamin B3 (niacin), colorants (E171, E172, E133), vitamin B8 (D-biotin).

The compounds contained in the composition for oral administration are advantageously used at doses or quantities for which an optimal effect is expected.

Said composition for oral administration may notably be presented in a form chosen from gel capsules, soft capsules, banded gel capsules, gels, dry or liquid emulsions, tablets, powders for dilution, oral ampules, suspensions, or oily suspensions.

The compositions according to the invention intended for oral administration are advantageously formulated in the form of foods or dietary supplements of nutraceutical compositions.

As such, in one particular embodiment, the compositions according to the invention are in the form of a dietary supplement.

Such formulations may comprise an ingestible substrate, the nature whereof is adapted according to the composition in question. As such, notably, tablets, capsules or pills, suspensions, oral supplements in dried form or oral supplements in liquid form, milk, yogurt, cheese, fermented milks, fermented milk-based products, ice-creams, cereal-based products or fermented cereal-based products, milk-based powders, or food products such as confectionery, chocolate, cereals are suitable as dietary substrates.

The formulations according to the invention may be produced by any routine method known to those skilled in the art for producing oral solutions, coated pills, gel capsules, emulsions, tablets to be swallowed or chewed, gel capsules, notably soft capsules, granules to be dissolved, syrups, solid or liquid foods and hydrogels suitable for controlled release, nutrition bars, powders, optionally compacted, suspensions of liquid solutions, confectionery, fermented milk, fermented cheeses, chewing gums, toothpastes or spray solutions.

The oral compositions may be presented either in anhydrous form, or in aqueous form according to the cosmetic indication.

A composition used within the scope of the invention may further be formulated with routine excipients and components for such oral compositions of dietary supplements, i.e. fatty and/or aqueous components, humectants, thickening agents, preservatives, texturants, bulking agents, anticaking agents, lubricants, flow agents, flavor film-coating and/or coating agents, colorants, notably pigments, and/or antioxidant agents.

The formulation agents and excipients for oral compositions, and particularly for dietary supplements, are known in this field and are not the subject of a detailed description herein. The formulation agents and excipients suitable for the invention may be chosen from among magnesium stearate, colloidal silica, starch, microcrystalline cellulose, hypromellose, and mixtures thereof.

The constituents of the composition for oral administration may be incorporated in any form of dietary supplements or enriched foods, for example nutrition bars or powders, optionally compacted. The powders may be diluted in water, soda, dairy products or soy derivatives, or be incorporated in nutrition bars.

The present invention relates to the use, notably cosmetic use, by oral route of a composition as described above to improve the cellulite appearance of the skin, particularly for maintaining and/or restoring the biomechanical properties of the skin, such as the properties in respect of stretchability, tone, firmness, suppleness, density and/or elasticity of the skin.

According to one embodiment, the aim of the use according to the invention is that of preventing or treating visual appearances associated with cellulite.

The use according to the invention is preferably intended for subjects, particularly women, of normal weight.

The recommended weight range for a subject may be estimated by calculating the BMI (Body Mass Index). The BMI is one of the indicators of normal weight which is calculated by dividing the subject's weight (in kg) by his/her height squared (in m). A BMI considered to be normal for a healthy human being is situated between 18.5 and 24.9 for an adult female and between 23 and 25 for an adult male. It consists obviously of a calculated indication and practitioners sometimes confirm this initial indication with further medical and biological examinations to confirm the actual physiological normal weight which may vary according to the subject's body type and other physiological parameters.

According to one embodiment, the use according to the invention relates to a subject, particularly a woman, having a normal BMI, i.e. between 18.5 and 28, particularly between 18.5 and 25.

According to a further embodiment, the use according to the invention relates to a subject not following a weight-reducing diet or slimming diet.

Preferably, the use according to the invention relates to a subject who is not in a weight loss phase.

The present invention also relates to a method, notably a cosmetic method, for improving the cellulite appearance of skin, said method comprising at least one step consisting of administering by oral route, simultaneously, separately or sequentially, to a subject, particularly a woman, in need thereof, at least one probiotic as defined above and at least one B group vitamin as defined above.

According to one embodiment, said at least one probiotic and said at least one B group vitamin are administered in the form of a single composition, particularly in the form of a composition as described above and particularly in the form of a dietary supplement as described above. According to one particularly preferred embodiment of the invention, said at least one probiotic and said at least one B group vitamin are administered in the form of a composition comprising 1x10⁵ to 1x10¹² cfu of *Lactobacillus rhamnosus* NCC 4007, 4 to 7 mg of a mixture of vitamin B3 and vitamin B8, 50 to 100 mg of FOS and 40 to 80 mg of inulin.

According to one particular embodiment, said administration of the composition is daily for at least 12 weeks.

Preferably, the method is characterized in that the administration is suitable for a daily administration of a combination of compounds comprising (i) 1x10⁵ cfu to 1x10¹² cfu of *Lactobacillus rhamnosus,* particularly *Lactobacillus rhamnosus* NCC 4007, or a mixture of probiotics, (ii) 4 to 7 mg of B group vitamin, particularly a mixture of vitamin B3 and vitamin B8, optionally (iii) 80 to 120 mg of grape seed extract, optionally (iv) 50 to 100 mg of FOS, and optionally (v) 40 to 80 mg of inulin.

According to one embodiment, the treated subject has a normal BMI, i.e. preferably between 18.5 and 25.

According to a further embodiment, said treated subject is not following a weight - reducing diet or slimming diet.

Preferably, the treated subject is not in a weight loss phase.

The present application will be illustrated in more detail by the figures and examples hereinafter.

### Figures:

Figure 1 represents the progression of the mean CSS score (Cellulite Severity Score) over the four months of supplementation as described in the example.
Figure 2 represents the follow-up of the CSS score between the final month of supplementation (month 4) and the following month, as described in the example.
Figure 3 represents the results of a survey among the women who perceived an effect of supplementation, assessing their perception of the effects thereof one month after discontinuation, as described in the example.

### Example:

The oral composition tested is as follows: Lactobacillus rhamnosus (LPR), fructo-oligosaccharides, chicory root extracts (Cichorium intybus), coating agent (hydroxypropylmethylcellulose), inulin extracted from chicory root (Cichorium intybus), anticaking agents (fatty acid magnesium salts, silicon dioxide); vitamin B3 (niacin), colorants (E171, E172, E133), vitamin B8 (D-biotin). It was tested for 4 months as a daily dose on 34 women between 20 and 45 years of age, not following a diet and having a normal BMI of 23.53 ± 1.94 kg/m² at the start of the study. It should be noted that the women taking part in this study did not have any significant weight variation during the study (weight of 64.34 ± 6.62 on average at the start of the study and 65.00 ± 6.59 on average at the end of the study, NS p=0.782), and thus a stable BMI of 23.9 kg/m² at the end of the study.

Each month, the degree of severity of the cellulite on their buttocks was evaluated using the CSS (Cellulite Severity score). This score is obtained by evaluating 5 parameters scored on a scale of 0 to 3 (Hexsel DM et al., A validated photonumeric cellulite severity scale. Journal of the European Academy of Dermatology and Venereology 2009; 23: 523-8).

These 5 parameters are:
- A: The number of visible depressions
- B: The depth of the visible depressions
- C: The clinical appearances of the lesions studied
- D: The presence of flaccidity, lack of firmness or slack skin
- E: The grade of cellulite

The CSS score is equal to the sum of the scores awarded to each parameter. A CSS score of 1 to 5 corresponds to a low degree of cellulite, 6 to 10 to a moderate degree of cellulite, 11 to 15 to a severe degree of cellulite.

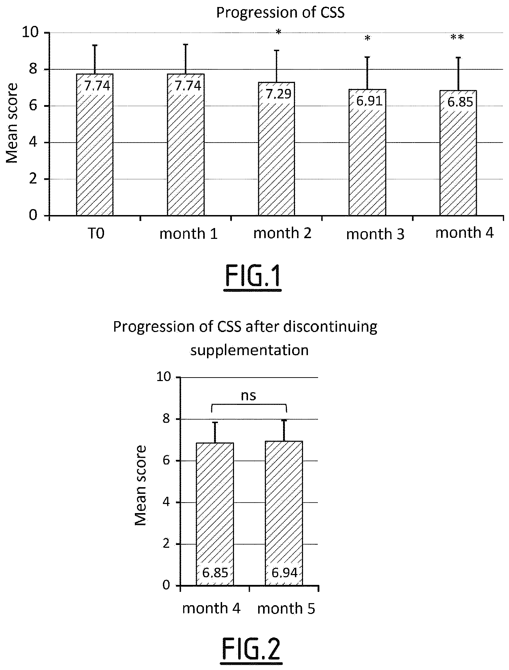

It is observed that taking the dietary supplement enables a significant reduction in the CSS score from the second month (figure 1), enabling after four months an average reduction of the CSS score by approximately 1 point. It is noted that this reduction persists further for one month after discontinuing the dietary supplement (figure 2).

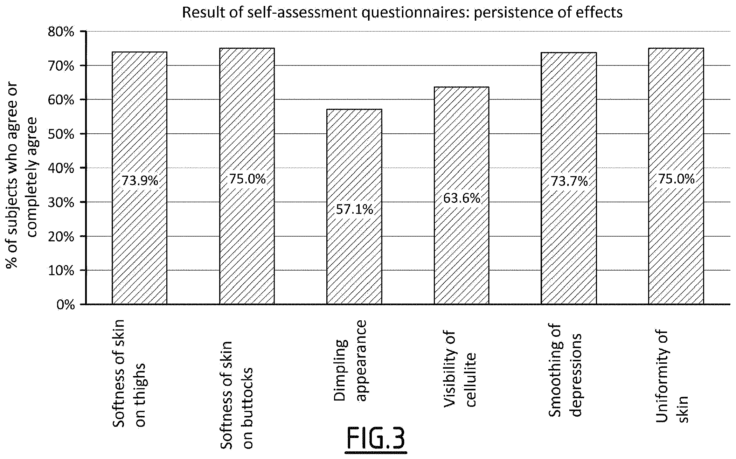

Furthermore, the women who observed improvements after four months were surveyed on the persistence of the effects of supplementation one month after the discontinuation thereof. The results are presented in figure 3. It is observed that a majority of the women surveyed were of the opinion that, one month after the end of supplementation, the effects on the softness of the skin, the dimpling appearance, the visibility of cellulite, the reduction in the depth of depressions and skin uniformity are still visible.

## Claims

1. Cosmetic non-therapeutic use by oral route of administration of a composition comprising at least one probiotic and at least one B group vitamin, for improving the cellulite appearance of skin in subjects not following a weight-reducing diet or slimming diet.

2. Use according to claim 1, wherein said at least one B group vitamin is vitamin B3 and/or vitamin B8.

3. Use according to any of claims 1 or 2, wherein said at least one probiotic is of the species *Lactobacillus rhamnosus.*

4. Use according to any of claims 1 to 3 for maintaining and/or restoring the biomechanical properties of skin.

5. Use according to any of claims 1 to 4 for maintaining and/or restoring the properties in respect of stretchability, tone, firmness, suppleness, density and/or elasticity of the skin.

6. Cosmetic non-therapeutic method for improving the cellulite appearance of skin, said method comprising at least one step consisting of administering by oral route, simultaneously, separately or sequentially, to a subject in need thereof wherein said subject is not following a weight-reducing diet or slimming diet, at least one probiotic and at least one B group vitamin.

7. Method according to claim 6, wherein said at least one probiotic and said at least one B group vitamin are administered in the form of a single composition and preferably in the form of a dietary supplement.

8. Method according to claim 7, wherein said composition is administered daily for a period of at least 12 weeks.

9. Method according to any of claims 6 to 8, wherein said subject is not in a weight loss phase.

10. Method according to any of claims 6 to 9, wherein said at least one probiotic is *Lactobacillus rhamnosus* NCC 4007 and said at least one B group vitamin is a mixture of vitamin B3 and vitamin B8.

11. Method according to claim 10, wherein said at least one probiotic and said at least one B group vitamin are administered in the form of a composition comprising 1x10⁵ to 1x10¹² cfu of *Lactobacillus rhamnosus* NCC 4007, 4 to 7 mg of a mixture of vitamin B3 and vitamin B8, 50 to 100 mg of FOS and 40 to 80 mg of inulin.

## Patentansprüche

1. Kosmetische, nichttherapeutische Anwendung durch orale Verabreichung einer Zusammensetzung, die mindestens ein Probiotikum und mindestens ein Vitamin der Gruppe B enthält, zum Verbessern des Auftretens von Cellulitis der Haut bei Subjekten, die nicht einer gewichtsreduzierenden Diät oder einer Schlankheitsdiät folgen.

2. Anwendung nach Anspruch 1, bei der das mindestens eine Vitamin der Gruppe B ein Vitamin B3 und/oder ein Vitamin B8 ist.

3. Anwendung nach einem der Ansprüche 1 oder 2, bei der das mindestens eine Probiotikum der Spezies Lactobacillus rhamnosus ist.

4. Anwendung nach einem der Ansprüche 1 bis 3 zum Aufrechterhalten und/oder Wiederherstellen der biomechanischen Eigenschaften der Haut.

5. Anwendung nach einem der Ansprüche 1 bis 4 zum Aufrechterhalten und/oder Wiederherstellen der Eigenschaften in Bezug auf Fähigkeit, Farbe, Geschmeidigkeit, Dichte und/oder Elastizität der Haut.

6. Kosmetisches, nichttherapeutisches Verfahren zum Verbessern der Cellulitiserscheinung der Haut, wobei das Verfahren mindestens einen Schritt umfasst, der darin besteht, oral, gleichzeitig, getrennt oder aufeinanderfolgend einer Person, die davon betroffen ist, mindestens ein Probiotikum und mindestens ein Vitamin der Gruppe B zu verabreichen, wenn diese Person nicht einer gewichtsreduzierenden Diät oder einer Schlankheitsdiät folgt.

7. Verfahren nach Anspruch 6, bei dem das mindestens eine Probiotikum und das mindestens eine Vitamin der Gruppe B in Form einer einzigen Zusammensetzung und vorzugsweise in Form eines Nahrungsergänzungsmittels verabreicht werden.

8. Verfahren nach Anspruch 7, bei dem die Zusammensetzung täglich für einen Zeitraum von mindestens 12 Wochen verabreicht wird.

9. Verfahren nach einem der Ansprüche 6 bis 8, bei dem die Person nicht in einer Gewichtsverlustphase ist.

10. Verfahren nach einem der Ansprüche 6 bis 9, bei dem das mindestens eine Probiotikum Lactobacillus rhamnosus NCC 4007 ist und das mindestens eine Vitamin der Gruppe B eine Mischung aus Vitamin B3 und Vitamin B8 ist.

11. Verfahren nach Anspruch 10, bei dem das mindestens eine Probiotikum und das mindestens eine Vitamin der Gruppe B in Form einer Zusammensetzung verabreicht wird, die 1x10⁵ bis 1x10¹² cfu des Lactobacillus rhamnosus NCC 4007, 4 bis 7 mg einer Mischung von Vitamin B3 und Vitamin B8, 50 bis 100 mg FOS und 40 bis 80 mg Inulin enthält.

## Revendications

1. Utilisation cosmétique non-thérapeutique par voie d'administration orale d'une composition comprenant au moins un probiotique et au moins une vitamine du groupe B, pour améliorer l'aspect cellulitique de la peau chez des individus ne suivant pas de régime amaigrissant ou amincissant.

2. Utilisation selon la revendication 1, dans laquelle ladite au moins une vitamine du groupe B est la vitamine B3 et/ou la vitamine B8.

3. Utilisation selon l'une des revendications 1 ou 2, dans laquelle ledit au moins un probiotique est de l'espèce *Lactobacillus rhamnosus.*

4. Utilisation selon l'une des revendications 1 à 3 pour maintenir et/ou restaurer les propriétés biomécaniques de la peau.

5. Utilisation selon l'une des revendications 1 à 4 pour maintenir et/ou restaurer les propriétés d'extensibilité, de tonicité, de fermeté, de souplesse, de densité et/ou d'élasticité de la peau.

6. Méthode cosmétique non-thérapeutique pour améliorer l'aspect cellulitique de la peau, ladite méthode comprenant au moins une étape consistant à administrer par voie orale, simultanément, séparément ou séquentiellement, à un individu en ayant besoin, dans laquelle ledit individu ne suit pas de régime amaigrissant ou amincissant, au moins un probiotique et au moins une vitamine du groupe B.

7. Méthode selon la revendication 6, dans laquelle ledit au moins un probiotique et ladite au moins une vitamine du groupe B sont administrés sous la forme d'une seule composition et de préférence sous forme d'un complément alimentaire.

8. Méthode selon la revendication 7, dans laquelle ladite composition est administrée quotidiennement pendant une durée d'au moins 12 semaines.

9. Méthode selon l'une des revendications 6 à 8, dans laquelle ledit individu n'est pas en période de perte de poids.

10. Méthode selon l'une des revendications 6 à 9, dans laquelle ledit au moins un probiotique est *Lactobacillus rhamnosus* NCC 4007 et ladite au moins une vitamine du groupe B est un mélange de vitamine B3 et de vitamine B8.

11. Méthode selon la revendication 10, dans laquelle ledit au moins un probiotique et ladite au moins une vitamine du groupe B sont administrés sous la forme d'une composition comprenant 1x10⁵ à 1x10¹² ufc de *Lactobacillus rhamnosus* NCC 4007, 4 à 7 mg d'un mélange de vitamine B3 et de vitamine B8, 50 à 100 mg de FOS, et 40 à 80 mg d'inuline.
